# EUROPEAN PATENT APPLICATION

(11) **EP 2 990 061 A1**
(43) Date of publication of application: **02.03.2016**
(21) Application number: 14182335.1
(22) Date of filing: 26.08.2014
(51) Int. Cl.: A61L 24/00, A61L 31/12, A61L 27/44, A61L 27/50

(54) **Radiopaque composition and preparation thereof**

(71) Applicant: Maastricht University, 6211 LK Maastricht (NL); Academisch Ziekenhuis Maastricht, 6229 HX Maastricht (NL)
(72) Inventor: Koole, L.H., 6211 LK Maastricht (NL); Saralidze, Ketie, 6211 LK Maastricht (NL)
(74) Representative: Renkema, Jaap

(57) **Abstract**

The invention relates to a radiopaque composition comprising at least one monomer, radiopaque filler particles and optionally a curing system for curing the monomer, wherein the radiopaque filler particles comprise a polymeric matrix having radiopaque material dispersed therein, and wherein the radiopaque filler particles have a particle size (d50) between 10 and 1000 pm determined by conventional transmission bright field light microscopy. The invention also relates to the use of the radiopaque composition as bone cement for orthopedic procedures. The radiopaque composition can be cured and also used for producing medical implants.

## Description

### Technical Field

The present invention generally relates to a radiopaque composition that can be used in clinical practice and can be readily viewed by common medical imaging modalities, such as X-ray fluoroscopy, computed tomography (CT) and magnetic resonance imaging (MRI). More particularly, the present invention relates to a radiopaque composition, comprising radiopaque filler particles, for implants and bone cements that can be used in orthopedic surgery for fixing and reinforcing bone structures, and for joining metallic, ceramic or polymeric implants to the skeleton. Furthermore, the present invention relates to the preparation of radiopaque filler particles, to the radiopaque filler particles and to a kit of parts comprising radiopaque filler particles, curing agent and monomer.

### Background art

Currently, the most commonly used compositions for applications in orthopedic surgery comprise acrylates, in particular methyl methacrylate (MMA), and its polymer, poly methyl methacrylate (PMMA). However, polymers such as polypropylene (PP), polyethylene (PE), acrylonitrile butadiene styrene (ABS), polyether ether ketone (PEEK), polyamide-imide (PAI), polyoxymethylene (POM), polyphenylsulfone (PPSU), polyethylenimine (PEI), ultra-high-molecular-weight polyethylene (UHMW-PE), polyurethane (PU), silicone, offer some other examples of compositions that are used in orthopedic surgery. In most cases the compositions are used to prepare bone cement or produce medical implants to reinforce and fix bone structures, and to join metallic, ceramic or polymeric implants to the skeleton.

The increasing prevalence of minimally invasive procedures, due to a variety of benefits relating to patient outcome and health care cost, has impacted the requirements for these compositions. This is mainly because the direct visual field is obscured and the visualization in most cases is achieved by other means than visible light, such as X-ray fluoroscopy, CT or MRI. In particular, in cases where X-ray fluoroscopy is used the previously described compositions do not provide the required properties for visualization, since they cannot easily be distinguished from other bodily tissues. This obligates that the visibility of these compositions is augmented. Currently, this is achieved either by adding radiopaque markers in case of medical implants or radiopaque material in case of bone cements. However, both of these solutions are subject to disadvantages.

For medical implants, for instance, the markers that aid in positioning do not allow complete visualization of the medical implant and therefore hamper accurate and efficient placement. Alternatively, metallic medical implants (e.g. titanium alloys, cobalt chrome alloys, stainless steel) are used, which are completely visible when imaged using X-ray fluoroscopy. However, these do not allow the simultaneous or subsequent use of MRI to visualize the surrounding soft tissues due to the susceptibility for magnetic interference, which may produce artifacts that blur the images.

For bone cements, for instance, complete visualization is not an issue, since the radiopaque material that is currently added, is dispersed throughout the composition. In bone cements this is most commonly achieved by adding barium sulfate to the composition, comprising polymeric particles (e.g. PMMA), a monomer (e.g. MMA) and a curing system, preferably comprising a free radical initiator (e.g. benzoyl peroxide) and an accelerator (e.g. N,N-Dimethyl para-toluidine). However, the disadvantage of this solution is that for several applications in orthopedic surgery, such as vertebroplasty, up to 30 wt. % of radiopaque material has to be used, because of the required level of radiopacity and the relatively poor radiopacifying properties of barium sulphate. These high concentrations may result in the formation of aggregates of barium sulfate and adversely affect various mechanical properties of the composition before and after surgery. For instance, prior to curing, it increases the viscosity of the mixture, which may adversely affect the mixing of the components and the homogeneity of the composition. Moreover, it hinders accurate and fast injection. Furthermore, after curing it negatively impacts tensile strength, compressive strength, flexural strength and fatigue life.

Another disadvantage of using barium sulphate as radiopaque material is that leaching of Ba²⁺ ions in situ has been shown to induce resorption of bone.

US4500658 describes using acrylic resin beads with a size of 10 to 100 µm, with 5 to 50 wt. % of radiopaque material, preferably barium sulfate, titanium dioxide , chromium oxide and zirconium dioxide, with a particle size of 0,1 to 20 µm. The disadvantage of using these radiopaque materials is that they offer limited radiopacity. Therefore, the composition of US4500685 still requires high amounts of radiopaque material (up to 50 wt. %) to achieve moderate radiopacity.

WO9918894 describes using radiopaque material such as barium sulfate, tungsten, tantalum, zirconium, platinum, gold, silver, stainless steel, titanium with a particle size between 120 and 2200 µm. The radiopaque material is provided as bare particles and thereby increases the risk of tissue exposure to these materials, which may adversely affect the health of the patient. For example, tantalum is not considered biocompatible and the use of tantalum for surgical implant applications is regulated

(e.g. ISO 13782). Furthermore, adding radiopaque material with a particle size between 120 and 2200 µm will hinder the injection of the composition through a syringe, which limits its usability during various minimally invasive procedures, such as (percutaneous) vertebroplasty, that require syringes, sometimes having a diameter of less than 2 mm, to apply the bone cement. Moreover, radiopaque material with a particle size between 120 and 2200 µm, and a density between 4,5 and 21,5 g/cm³ may easily sediment in a composition with a density between 0.9 and 1.2 g/cm³ and thereby adversely affect the mechanical properties and visibility of the complete composition using X-ray fluoroscopy. Additionally, using bare radiopaque material of the indicated size range may adversely affect the mechanical properties of the cured composition by producing stress concentrations at the interface between the radiopaque material and the cured resin. In turn, this can cause fractures in the cured composition and as a consequence necessitate additional surgery.

WO02096474 describes polymer coated radiopaque material comprising radiopaque material such as tantalum and tungsten. The polymer coating is provided to eliminate the exposure of the radiopaque material to oxygen and thereby reduces the risk of adverse effects on patient health. However, the use of polymer coated radiopaque material with a high density such as tantalum (16,9 g/cm³) and tungsten (19,3 g/cm³) that makes up between 70 and 95 wt. % of the coated radiopaque material, has the disadvantage that the density of the polymer coated radiopaque material will also be high (approximately between 2,7 and 12,0 g/cm³) compared to bone cement (approximately between 0.9 and 1.2 g/cm³), since this can result in the sedimentation of the polymer coated radiopaque material and reduce the homogeneity of the composition. This negatively affects the visualization of the material. Furthermore, this has an adverse impact on the mechanical properties of the composition before, during and after curing. In addition to these disadvantages, the use of these non-biocompatible radiopaque materials poses a risk that should always be avoided in clinical practice, since errors or material failure that may result in damaging tissue exposure can never be fully excluded.

In general, compositions used in orthopedic surgery suffer from several disadvantages and limitations closely related to the intrinsic properties of these compositions regarding visibility during procedures that utilize imaging modalities, such as X-ray fluoroscopy, CT and MRI. Current solutions to optimize their visibility adversely affect the mechanical properties before curing and after curing. Improving the visibility of these compositions, without adversely affecting the material properties, is pivotal to improve and increase the applicability of minimally invasive surgical interventions in orthopedics.

### objects of the invention

The object of the invention is to provide a radiopaque composition with an adjustable viscosity that has improved visibility when used in combination with various imaging techniques, such as X-ray fluoroscopy, CT and MRI. Furthermore, it is an object of the invention to improve the mechanical properties of the radiopaque compositions before and during curing, such as reducing the viscosity, and optionally after curing, such as higher tensile, compressive and flexural strength and prolonged fatigue life. An additional object of the invention is to provide a freedom of choice regarding the viscosity before and during curing that are best suited for the application. The overall goal of these enhancements is to improve current surgical procedures, to enable advancements in minimally invasive surgery and to enable follow up patient care.

### Summary of the invention

The invention relates to a radiopaque composition comprising at least one monomer, radiopaque filler particles and optionally a curing system for curing the monomer, wherein the radiopaque filler particles comprise a polymeric matrix having radiopaque material dispersed therein, and wherein the radiopaque filler particles have a particle size (d50) between 10 and 1000 µm determined by conventional transmission bright field light microscopy.

The advantage of using radiopaque filler particles that have radiopaque material dispersed therein is that it enables the use of radiopaque materials with a higher radiopacity (e.g. materials with a higher atomic mass, such as gold) or other desired properties, that otherwise are not suitable, for instance because of the risk of sedimentation of the higher density materials or the risk of exposing tissue to the radiopaque material itself or leachable components of the radiopaque material. Furthermore, using radiopaque materials with a higher atomic mass reduces the required volume percentage of radiopaque material with respect to the entire composition to obtain a specific level of radiopacity, which improves the mechanical properties of the composition after curing. Moreover, using radiopaque filler particles that have radiopaque material dispersed therein eliminates the necessity of separately adding radiopaque materials to the composition, which has several benefits regarding the mechanical properties of the composition before and after curing. For instance before and during curing, the increase of viscosity as a result of supplementing the composition with radiopaque material such as barium sulphate is eliminated. The resulting lower viscosity is advantageous with respect to injecting the bone cement, because it reduces the required pressure and thereby improves the speed and accuracy of the surgical procedure. Furthermore, it improves the homogeneous distribution of radiopaque material within the composition, which is related to improved visibility and mechanical properties.

For these reasons, eliminating the necessity of separately adding radiopaque materials to the composition to alter the radiopacity of the compound is no longer directly related to altering the mechanical properties of the composition. Additionally, altering the size and amount of the radiopaque filler particles will allow relatively simple optimizations regarding the required mechanical properties for specific applications. Furthermore, it simplifies the procedure of preparing the composition, because there is no need to add supplementary radiopaque material, which in turn reduces the chance of error and issues relating to the sterility of the composition.

Surprisingly, the present invention allows the use of both X-ray fluoroscopy, CT and MRI to visualize hard tissue such as bone, soft tissue such as the nerves and the implant both during and after the surgical procedure, which enables the further refinement and optimization of these interventions. Another surprising result is that the present invention enables the preparation of a low viscous bone cement that has a high radiopacity.

### Detailed Description of the invention

The invention relates to a radiopaque composition comprising at least one monomer, radiopaque filler particles and optionally a curing system for curing the monomer, wherein the radiopaque filler particles comprise a polymeric matrix having radiopaque material dispersed therein, wherein the radiopaque filler particles have an average particle size (d50) between 10 and 1000 µm determined by conventional transmission bright field light microscopy and wherein the radiopaque filler particles comprise a polymer matrix and dispersed radiopaque material.

Various types of monomers are currently used in clinical practice and specifically for orthopedic surgery. Examples are monomers, that make up the monomeric subunits of polymers, belonging to the group of acrylates, urethanes, alkenes, silicones. All of these are suitable for the present invention, but preferably the monomers a chosen from the group consisting of acrylates and methacrylates; most preferably the monomer is methyl methacrylate (MMA).

The radiopaque composition comprises between 20 and 70 wt. % of monomer, preferably between 25 and 50 wt. %, more preferably between 30 and 40 wt. %.

The polymer matrix of the radiopaque filler particles is preferably prepared from monomeric units belonging to the same group as the monomer of the radiopaque composition, but may also belong to a different group of polymers or be a copolymer. Preferably PMMA is used for the polymer matrix.

The radiopaque filler particles comprise between 70 and 99,5 wt. % of polymer matrix, preferably between 80 and 97 wt. %, more preferably between 85 and 95 wt. %.

Furthermore, the radiopaque filler particles comprise between 0,5 and 30 wt. % of radiopaque material, preferably between 3 and 20 wt. %, more preferably between 5 and 15 wt. %.

The radiopaque filler particles preferably have an average particle size (d50) between 10 µm and 1000 µm as can be determined by conventional transmission bright field light microscopy.

The radiopaque filler particles preferably have a spherical or oval shape with a maximal aspect ratio of 10.

The radiopaque composition comprises between 30 and 80 wt. % of radiopaque filler particles, preferably between 50 and 75 wt. %, more preferably between 60 and 70 wt. %.

The radiopaque material is essentially composed of elements with a high atomic mass, preferably equal to or higher than 140 Dalton. In particular, a noble metal like gold is suited to be used as a radiopaque material in medical applications, because of its high atomic mass, chemical inertness and non-magnetic properties.

The radiopaque material preferably has a particle size (d50) between 0,5 µm and 5 µm as can be determined by electron microscopy.

The radiopaque composition comprises between 0,15and 24 wt. % of radiopaque material, preferably between 1,5 and 15 wt. %, more preferably between 3 and 10,5 wt. %.

Optionally the radiopaque composition may contain other filler particles, which do not contain radiopaque material. Examples of other filler particles are polymer particles, like PMMA particles.

Optionally the radiopaque composition comprises a curing system, preferably comprising an initiator and an accelerator, which are responsible for curing the radiopaque composition. The initiator initiates the polymerization reaction by providing a free-radical and the accelerator may speed up the curing process.

Examples of initiators that can be used are tri-n-butylborane, benzoyl peroxide, campherquinone, phenylpropanedoine, 2,4,6-trimethylbenzoyl-diphenyl-phosphineoxide, preferably benzoyl peroxide is used.

Examples of accelerators that can be used are 4-N,N-(dimethylamino)phenethanol, acryloyl-N-phenylpiperazine, methacryloyl-N-phenylpiperazine, dihydroxypropyl-p-toluidine, N,N-Dimethyl para-toluidine as a stabilizer, 4-dimethylamino phenetyl alcohol, 2-5-dimethylhexane-2-5-hydroperoxide, 4-dimethylaminobenzyl alcohol, 4-dimethylaminobenzyl methacrylate , preferably 4-N,N-(dimethylamino)phenethanol is used.

The radiopaque composition comprises preferably between 0,1 and 10 wt. % of curing system, comprising between 10 and 99,5 wt. % of initiator, preferably between 50 and 95 wt. %, more preferably between 60 and 90 wt. % and between 0,5 and 90 wt. % of accelerator, preferably between 5 and 50 wt. %, more preferably between 10 and 40 wt. %.

The invention further relates to a cured radiopaque composition that is prepared by curing the radiopaque composition according to the invention. As a result, the cured radiopaque composition comprises a continuous and dispersed phase, wherein the continuous phase does not comprise radiopaque material, and wherein the dispersed phase contains radiopaque material.

The continuous phase is essentially composed of a polymer matrix A and the dispersed phase comprises between 70 and 99,5 wt. % of a polymer matrix B and between 0,5 and 30 wt. % of radiopaque material.

The polymer matrix A of the continuous and the polymer matrix B of the dispersed phase can be the same or different. Preferably the polymer matrix A of the continuous phase is the same as the polymer matrix B of the dispersed phase and is made up of polymers or copolymers belonging to the group of acrylates, urethanes, alkenes or silicones. Preferably PMMA is used as polymer matrix A and B.

The cured radiopaque composition comprises 20 and 70 wt. % of continuous phase and between 30 and 80 wt. % of dispersed phase.

A cured radiopaque composition is ideally suited to produce a shaped cured radiopaque composition. This can be achieved by various additive and reductive manufacturing methods known to people skilled in the art. Injection molding, 3D-printing, extrusion molding, selective laser sintering and stereolithography are just some examples of additive manufacturing methods suitable for obtaining the required shape. Reductive manufacturing, where the removal of material is used to acquire the required shape, can be achieved by machining, milling, drilling, high pressure water jet cutting, laser cutting, etching or any suitable material removal technique available.

The invention further relates to a method for preparing radiopaque filler particles, said method comprising the steps of:
a) providing an aqueous solution comprising detergent;
b) providing a suspension comprising monomer, radiopaque material and, curing system and polymer filler particles;
c) curing the suspension by adding the suspension to the aqueous solution while stirring to obtain radiopaque filler particles;
d) Isolation of the filler particles.

A preferred method for preparing radiopaque filler particles according to the invention, comprises the steps of:
a) providing an aqueous solution comprising 0,1-10 wt. % poly(vinyl alcohol), 0,1-10 wt. % poly(ethylene glycol), 0,1-10 wt. % poly(vinyl pyrrolidinone),
b) providing a suspension comprising 10-80 wt. % monomer, 1-60 wt. % radiopaque material essentially composed of gold, 1-10 wt. % initiator, 1-10 wt. % tetra-ethylene glycol dimethacrylate, 0,1-5 wt. % accelerator, 5-25 wt. % NaCl and 15-60 wt. % polymer filler particles of a molecular weight between 50.000 and 250.000 Da,
c) heating the aqueous solution to a temperature between 50 and 95 degrees Celsius,
d) adding the suspension while stirring the aqueous solution at 500-2000 rpm,
e) curing for 1,5-10 hours,
f) stop stirring to allow the radiopaque filler particles to precipitate,
g) decanting the supernatant,
h) washing the radiopaque filler particles with water,
i) selecting the correctly sized radiopaque filler particles by sieving,
j) freezing the correctly sized radiopaque filler particles between -20 and -180 degrees Celsius and
k) lyophilizing the frozen radiopaque filler particles.

The radiopaque composition of the present invention can be used for various applications in clinical practice and orthopedic procedures in particular.

The radiopaque composition, for instance, is ideally suited to be utilized as bone cement for orthopedic procedures. There are various uses for bone cement in orthopedic surgery. For instance, it may be used to fixing implants, but it is also used for stabilizing bone structures.

The radiopaque composition is particularly suited for procedures where there are many requirements regarding the properties of the radiopaque composition, such as vertebroplasty. During these procedures it is very important to have appropriate properties (e.g. mechanical, radiopacity) before and during curing, to enable a fast and accurate application of the bone cement through a syringe without risking leakage of the bone cement into the surrounding tissue, and after curing, to have good mechanical properties to avoid follow up surgery.

The cured radiopaque composition is ideally suited for producing medical implants, for instance for application in surgical interventions on the spine.

The cured radiopaque composition is particularly suited for implants used in the upper spinal region, such as interbody fusion cages. This is mainly because in this region both the hard tissues (e.g. bone) and soft tissues (e.g. nervous system) are of interest and the radiopaque composition can be imaged using X-ray fluoroscopy, CT and MRI, which are suitable for imaging hard and soft tissue.

The invention further relates to a kit of parts comprising radiopaque filler particles, curing agent and monomer, wherein the radiopaque filler particles comprise polymeric matrix B and radiopaque material, wherein the radiopaque filler particles have a particle size between 10 µm and 1000 µm determined by conventional transmission bright field light microscopy, wherein the radiopaque material has a particle size (d50) between 0,5 µm and 5 µm determined by electron microscopy and wherein the radiopaque material comprises elements having an atomic mass equal to or higher than 140 Da, in particular gold.

### Description of figures

Figure 1 shows particles with and without radiopaque material.
Figure 2 shows a radiopaque composition having filler particles that contain radiopaque material
Figure 3 shows a radiopaque composition comprising radiopaque filler particles and other filler particles that do not have radiopaque material.
Figure 4 shows placement of a vertebrae in the bundle of the fluoroscopic guidance system (C-arm). One trocar has been inserted (left pedicle).
Figure 5 shows detailed image of the trocar.
Figure 6 shows example of a real-time X-ray image, showing correct placement of the trocar's tip at the center of the vertebra.
Figure 7 shows scanning electron micrographs of gold spheres with NaCl after washing; note that most of the particles are spherical. Some deviating geometries are also present (e.g. peanut-shaped particles apparently resulting from collision and fusion during suspension polymerization).
Figure 8 shows a typical example of a cylindrical gold-containing cement specimen, with dimensions: height = diameter = 6.0 mm; these samples were used in our uniaxial compression measurements. Cement specimens of the materials PMMA (control cement without any contrast agent), White (control cement (commercial)), and W2 (gold-containing cement). The specimens were compressed to 50 % of their original height, and this resulted in permanent deformation, as is shown on the left side of each photograph.
Figure 9 shows a typical stress/strain curve of a uniaxial compression test. Such a curve was measured for every cement specimen. The most essential material parameter is the Compression Modulus. This parameter is readily abstracted from the curve, as it is the slope of the curve in the linear range, which refers to small displacement and elastic behavior.

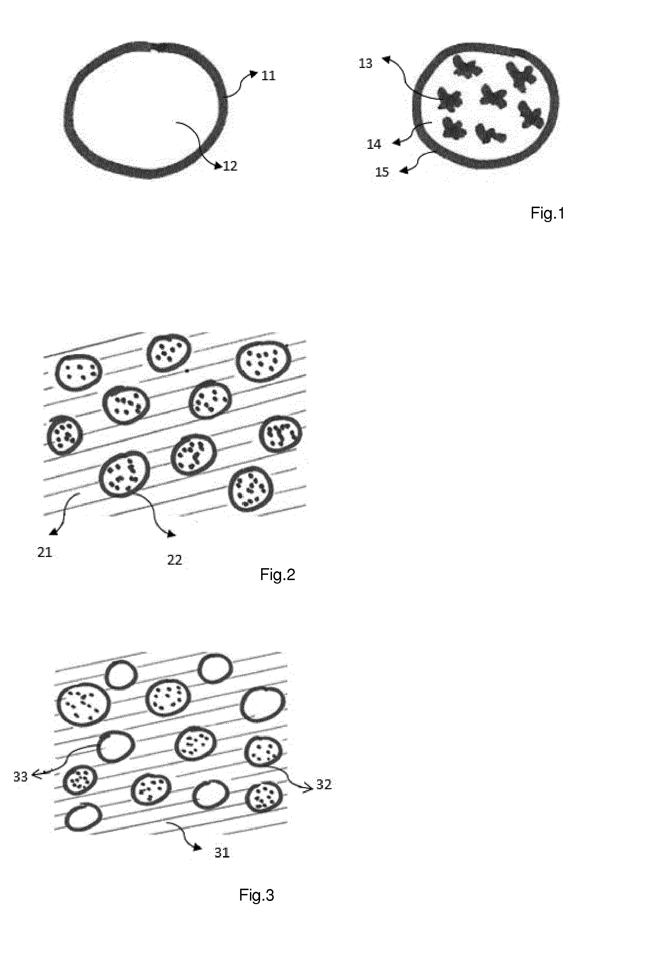

Referring to Fig. 1, the filler particle (11) currently mostly used in bone cement comprise polymer (12). The present invention relates to filler particles (15) that comprise polymer (14) and radiopaque material (13) dispersed therein.

Referring to Fig. 2, the radiopaque composition or cured radiopaque composition comprises radiopaque filler particles (22) dispersed in a continuous phase (21) comprising monomer or polymer, respectively.

Referring to Fig. 3, the radiopaque composition or cured radiopaque composition comprises radiopaque filler particles (32) and filler particles without radiopaque material (33) dispersed in a continuous phase (31) comprising monomer or polymer, respectively.

### EXAMPLES

### Synthesis of gold-containing PMMA microspheres:

An aqueous solution (150 mL) of poly(vinyl alcohol) (3.5 g, Mw = 86,000), poly(ethylene glycol (Mw = 1,000) and poly(N-vinyl pyrrolidinone) (Mw = 58,000) was prepared in a 250-mL round bottom flask. The solution was magnetically stirred (1100 rpm) and heated to 86-87 °C. A suspension of methyl methacrylate (15.0 g), gold microparticles (1.00 g), benzoyl peroxide (500 mg), tetraethyleneglycol dimethacrylate (410 mg), N,N-dimethyl toluidine (90 mg), NaCl (2.5 g), and PMMA (3.24 g, Mw= 120,000) was prepared in a 100-mL Erlenmeyer flask. The viscous slurry was poured slowly into the hot and stirred aqueous phase. Heating and stirring were continued for 4 h. Formation of microspheres was confirmed by taking an aliquot and examining this by light microscopy. Then, the heat source was turned off, and the flask was allowed to cool to room temperature. After several hours, stirring was stopped and the microspheres were allowed to settle on the bottom of the flask. The microspheres were isolated by filtration, washed with water (5 times), dried in air, and lyophilized. The microspheres were size-sorted by sieving in two classes: 300 - 600 µm diameter, and < 300 µm diameter, using calibrated circular sieves with a diameter of 20 cm (Retsch, Germany).

### Preparation of the gold-containing bone cement:

Gold-containing microspheres with diameter in the range 300-600 µm (4.00 g) were transferred into a bowl of aluminium foil, and 110 mg of benzoyl peroxide was added. A stock solution of methyl methacrylate and N,N-dimethyl toluidine (40.0 : 0.5 g:g) was made in a closed 100-mL Erlenmeyer flask (in the fumehood). Part of this solution (4.00 g) was transferred into the aluminium bowl, using a glass pipette. The contents of the bowl were gently mixed with a spatula for several minutes, and formation of a dough was noted.

The cement dough was used to manufacture material samples for uniaxial compression analysis, for experiments to characterize the material's in vitro cell compatibility and for analysis by scanning electron microscopy. The dough was inserted carefully into four different 5-mL polypropylene syringes from which the plunger was removed. The inner diameter of the syringes was 6 mm, and appr. 2 g of the cement dough was inserted into each syringe. The plunger was re-inserted, and the dough was manipulated carefully to remove enclosed air bubbles as far as possible. The filled syringes were laid horizontally in the fume hood. After approximately 12 min, the syringes became hot (> 80 °C), showing that the polymerization reaction took place. The filled syringes were left untouched for 24 h. A cement rod was carefully removed from each syringe, using a Stanley knife. Material samples, either cylinders of 6.0 mm height and 6.0 mm diameter (for uniaxial compression experiments and X-ray contrast measurements), or cylinders of 1.0 mm height and 6.0 mm diameter (for use in contact with cells or for use in microscopic analysis) were obtained by machining.

### Scanning electron microscopy:

Scanning electron microspcopy was performed with a Philips XL-30 instrument, at an accelerating voltage of 10 keV. Images were recorded in the standard mode or in the backscattered mode. Prior to examination, samples were gently polished and sputter-coated with gold (for direct examination), or carbon (for imaging in the backscattered mode). For imaging of microspheres in the backscattered mode, the particles were first immersed in an epoxy resin. After curing of the resin, slices were cut. The surfaces were polished and sputter-coated with carbon, prior to examination.

### Uniaxial compression:

These experiments were carried out on an Instron Automated Materials Testing System (LX Model) equipped with 10kN loading cell. The cylinder cement samples with height = 6.0 mm and diameter = 6.0 mm were used. Samples were tested at the compression rate 1 mm/min (n = 8-10). Compression was stopped at 50 % compression (height = 3.0 mm). The modulus was calculated from the slope of the linear part of the stress/strain diagram

### Cement injections into human vertebrae with simulated compression fractures:

Three fresh cadaveric thoracolumbar spines (Th6-L5, two female, one male, average donor age 89 (86-91) years), were made available for these experiments. The spines were obtained through the Department of Anatomy, Maastricht University Medical Centre Plus, and were donated for medical research. The spines were kept frozen (-20 °C) in sealed plastic bags. First, the spines were inspected with dual energy radiograph absorptiometry (DEXA) and computed tomography (CT) to find possible fractures, and to determine the anterior, posterior and midline planes of each vertebral body. After complete thawing (20 °C), the spines were disarticulated and excised of soft tissue. Posterior elements were removed to facilitate mechanical testing. The vertebrae were separated, and were excluded. Within each spine, consecutive vertebrae were considered paired specimens. So, for example, T6 and T7 formed one pair, as well as L4 and L5, etc. Thirty vertebrae were obtained and assigned into one of two groups: (i), gold-containing cement and commercial (BaSO4-containing) cement. The commercial cement was the Vertaplex HV Bone Cement, manufactured by Stryker, Michigan, USA); note that the powder part contains 30 % (by mass) of BaSO4. The superior vertebral body within each pair was assigned to one of the two experimental groups, and the inferior vertebra to the opposite group. Between the 2 groups, the assignment of subsequent pairs was alternated to avoid potential bias.

The anteroposterior and mediolateral diameters and anterior, posterior and lateral heights of each vertebra were measured three times using digital calipers (Mitutoyo MTI, Corp., Aurora, IL); these measurements were averaged to determine the representative measures of each vertebral body. Each vertebra was floated in a sealed plastic bag in a water bath maintained at 37°C for at least 1 h prior to testing. Initial stiffness was defined as the average slope on the load-displacement curve. Strength was defined as the inflection point on the load-displacement curve. By means of a ball-and-socket system pure axial compression was ensured to simulate a compression fracture. Compression was applied at a rate of 5 mm/min to the superior impression, until the anterior height of the vertebrae was decreased by 25%, which was defined as the compression fracture model (failure load).

After creation of the simulated compression fracture in each of the vertebrae one of the two cements was carefully injected. Through each pedicle, a 12-gauge vertebroplasty injection needle was inserted under biplanar fluoroscopic control. The assigned cement was prepared as described above (for the gold-containing cement; 19.76 mL liquid, and 19.47 g of powder were mixed), or as prescribed by the manufacturer (commercial BaSO4 cement). Cements were hand-mixed for 300 s (timed on stopwatch), and then transferred into moved into the syringes.

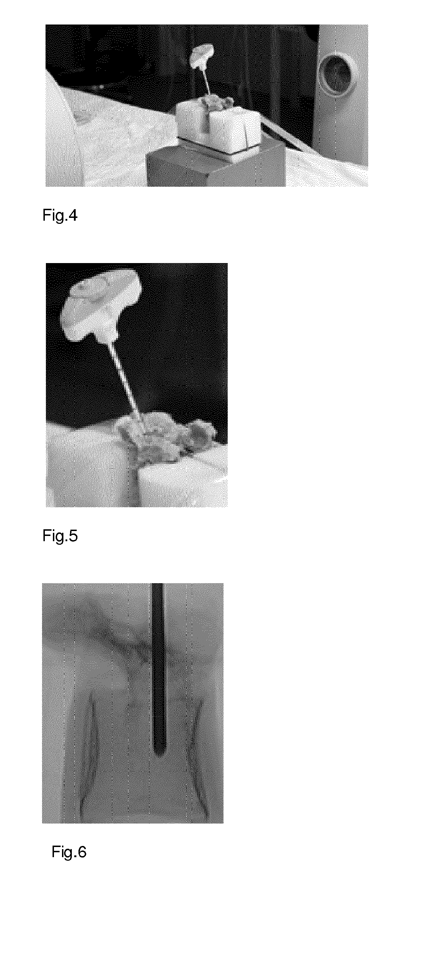

The cements were injected bipedicularly under lateral C-arm fluoroscopic control (see Figure 4, 5 and 6) by PW, who is an experienced spine surgeon. Injection was stopped if (pending) extravasation was observed. At thoracic levels a maximum volume of 6 mL was injected through each needle into the interior of the vertebra and a maximum volume of 8 mL was injected at lumbar levels. In order to mimic body temperature the vertebrae were sealed in plastic bags and floated in a bath maintained at 37°C for 24 hours after cement augmentation.

The vertebral specimens were compressed again according to the initial crush protocol until failure, and post-treatment stiffness and strength were determined as before. Some of the vertebrae were inspected with high-resolution CT imaging (Xtreme CT). Images were recorded at the following time points: before initial compression, after initial compression, and after cement augmentation. The last image series allowed us to characterize determine filling and distribution of the particular cement in the vertebral bodies. The force-displacement curves acquired at the compression tests were used to determine failure load and stiffness of each vertebral body prior to and after cement augmentation. A non-parametric statistical hypothesis test (Wilcoxon signed-rank test) was used to characterize the differences between the 2 experimental groups.

### Results and Discussion

### Microsphere synthesis:

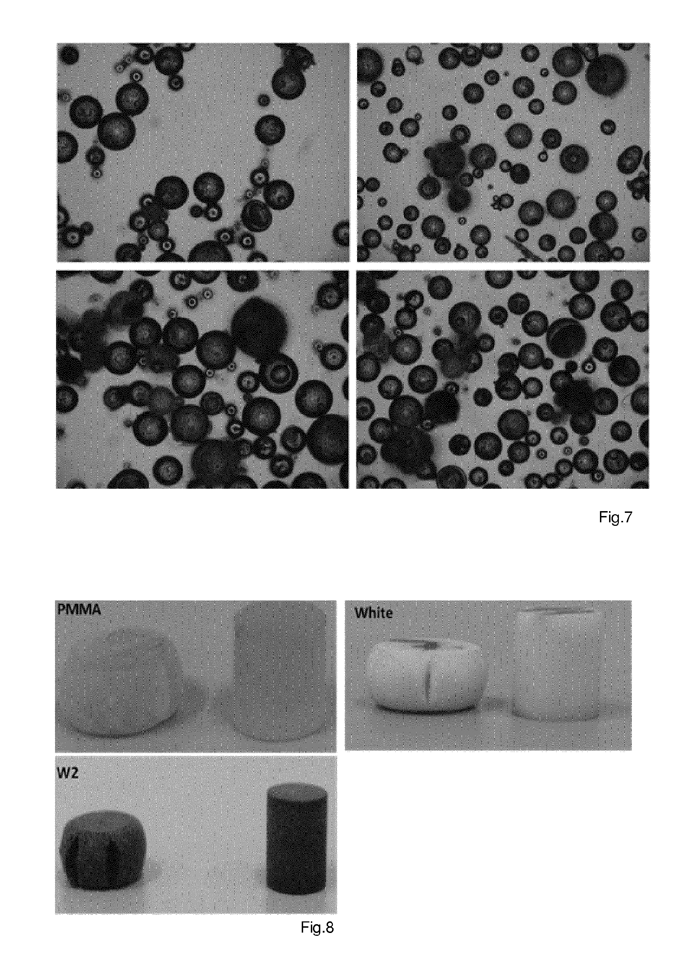

Preparation of the gold-loaded microspheres proceeded smoothly, reproducibly, and with satisfactory yields (> 80 %). The resulting particles were sieved and those within the diameter range 200-400 µm were collected. In other words, these were the particles that passed a sieve with mesh-size 400 µm, but did not pass a sieve with mesh size 200 µm. Figure 7 shows scanning electron micrographs of these particles; note that most of the particles are spherical. Some deviating geometries are also present (e.g. peanut-shaped particles apparently resulting from collision and fusion during suspension polymerization).

### Manufacture of cement specimens:

Hand-mixing of the gold-containing particles combined with MMA monomer also proceeded smoothly. After several minutes, a soft and pliable dough was obtained which could be transferred easily and without inclusion of air bubbles into 5-mL syringes from which the plunger was removed. Such syringes were used in order to prepare cement samples as straight cylinders with a defined diameter. After 10-15 minutes, a clear rise in temperature was noted, obviously due to exothermic polymerization of MMA. After 24 h, the cement cylinders were isolated from the syringes. Samples for uniaxial compression, scanning electron microscopy, evaluation of X-ray contrast, or for in vitro tests of cyto-compatibility were manufactured from these rods in our mechanical workshop. Analogously, specimens were prepared from the SimplexR cement for vertebroplasty and from PMMA (i.e. the latter cement did not contain gold or barium sulfate nor any other contrast agent).

Figure 8 shows a typical example of a cylindrical gold-containing cement specimen, with dimensions: height = diameter = 6.0 mm; these samples were used in our uniaxial compression measurements.

### Uniaxial compression:

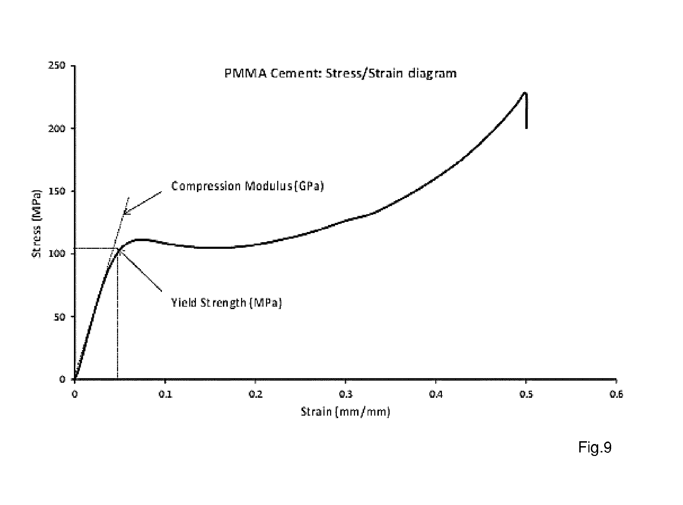

Figure 9 shows a typical stress/strain curve. The most important parameter, the elastic modulus (E), was abstracted from the linear part of each stress-strain curve, i.e. at displacement < 10 %. Experiments were done in 6-fold (for PMMA) or in 8-fold (for the commercial and gold-containing cements). The resulting data are compiled in Table 1.

The data reveal that the gold-containing cement is somewhat softer than the commercial cement and the PMMA control.

| **Table 1. Results from uniaxial compression tests on the gold-containing cement, its commercial counterpart, and contrast-free (radiolucent) PMMA cement.** | | | |
|---|---|---|---|
| | Elastic modulus (compression, MPA) | | |
| | PMMA | Commercial cement | Gold cement |
| | 2217 | 2410 | 2319 |
| | 2559 | 2426 | 1764 |
| | 2415 | 2900 | 2353 |
| | 2161 | 2450 | 1743 |
| | 2611 | 2331 | 2369 |
| | 2676 | 2807 | 2339 |
| | 2594 | 2781 | 2320 |
| | 2768 | 2803 | 2400 |
| | | 2840 | 2223 |
| | | 2886 | 1930 |
| | | | |
| Mean (MPa) | 2500 | 2663 | 2176 |
| Standard deviation (MPa) | 217 | 228 | 260 |

## Claims

1. A radiopaque composition comprising at least one monomer, radiopaque filler particles and optionally a curing system for curing the monomer, wherein the radiopaque filler particles comprise a polymeric matrix having radiopaque material dispersed therein, and wherein the radiopaque filler particles have a particle size (d50) between 10 and 1000 µm determined by conventional transmission bright field light microscopy.

2. The radiopaque composition according to claim 1, wherein the monomer is methyl methacrylate (MMA).

3. The radiopaque composition according to anyone of claims 1-2, wherein the composition comprises between 20 and 70 wt. % of monomer, preferably between 25 and 50 wt. %.

4. The radiopaque composition according to anyone claims 1-3, wherein the polymer matrix is poly methyl methacrylate (PMMA).

5. The radiopaque composition according to anyone of claims 1-4, wherein the radiopaque filler particles comprise between 70 and 99,5 wt. % of polymer matrix.

6. The radiopaque composition according to anyone of claims 1-5, wherein the radiopaque material is essentially composed of elements with a high atomic mass, preferably equal to or higher than 140 Dalton, preferably gold.

7. The radiopaque composition according to anyone of claims 1-6, wherein the radiopaque composition comprises preferably between 0,1 and 10 wt. % of curing system, comprising between 10 and 99,5 wt. % of initiator and between 0,5 and 90 wt. % of accelerator.

8. A cured radiopaque composition that is prepared by curing the radiopaque composition according to anyone of claims 1-7.

9. Use of the radiopaque composition according to anyone of claims 1-7 as bone cement for orthopedic procedures.

10. Use of the cured radiopaque composition according to claim 8 for producing medical implants.

11. A kit of parts comprising radiopaque filler particles, curing agent and monomer, wherein the radiopaque filler particles comprise polymeric matrix and radiopaque material, wherein the radiopaque filler particles have a particle size (d50) between 10 µm and 1000 µm determined by conventional transmission bright field light microscopy, wherein the radiopaque filler particles comprise a polymer matrix and dispersed radiopaque material and wherein the radiopaque material comprises elements having an atomic mass equal to or higher than 140 Da, in particular gold.

12. Radiopaque filler particle having a particle size (d50) between 10 and 1000 µm determined by conventional transmission bright field light microscopy, comprising between 70 and 99.5 wt% of a polymer matrix, and between 0.5 and 30 wt% of radiopaque material, wherein the radiopaque material is dispersed in the polymer matrix.

13. Radiopaque filler particle according to claim 12 comprising radiopaque material essentially composed of elements with a high atomic mass, preferably equal to or higher than 140 Dalton, preferably gold.

14. A method for preparing radiopaque filler particles according to anyone of claims 12-13, said method comprising the steps of:
i. providing an aqueous solution comprising detergent;
ii. providing a suspension comprising monomer, radiopaque material, curing system and polymer filler particles;
iii. curing the suspension by adding the suspension to the aqueous solution while stirring to obtain radiopaque filler particles;
iv. Isolation of the filler particles.

15. A method for preparing radiopaque filler particles according to claim 14, said method comprising the steps of:
i. providing an aqueous solution comprising 0,1-10 wt. % poly(vinyl alcohol), 0,1-10 wt. % poly(ethylene glycol), 0,1-10 wt. % poly(vinyl pyrrolidinone),
ii. providing a suspension comprising 10-80 wt. % monomer, 1-60 wt. % radiopaque material essentially composed of gold, 1-10 wt. % initiator, 1-10 wt. % tetra-ethylene glycol dimethacrylate, 0,1-5 wt. % accelerator, 5-25 wt. % NaCl and 15-60 wt. % polymer matrix of a molecular weight between 50.000 and 250.000 Da,
iii. heating the aqueous solution to a temperature between 50 and 95 degrees Celsius,
iv. adding the suspension while stirring the aqueous solution at 500-2000 rpm,
v. curing for 1,5-10 hours,
vi. stop stirring to allow the radiopaque filler particles to precipitate,
vii. decanting the supernatant,
viii. washing the radiopaque filler particles with water,
ix. selecting the correctly sized radiopaque filler particles by sieving,
x. freezing the correctly sized radiopaque filler particles between - 20 and -180 degrees Celsius and
xi. lyophilizing the frozen radiopaque filler particlesproviding an aqueous solution comprising 0,1-10 wt. % poly(vinyl alcohol), 0,1-10 wt. % poly(ethylene glycol), 0,1-10 wt. % poly(vinyl pyrrolidinone),
